# EUROPEAN PATENT APPLICATION

(11) **EP 4 186 929 A1**
(43) Date of publication of application: **31.05.2023**
(21) Application number: 21851546.8
(22) Date of filing: 19.08.2021
(51) Int. Cl.: C07K 19/00, C12N 15/62, C12N 5/10, A61K 39/00, A61K 35/17, A61P 35/00

(54) **NOVEL CO-STIMULATORY DOMAIN AND USES THEREOF**

(71) Applicant: Nanjing Bioheng Biotech Co., Ltd, Nanjing, Jiangsu 210061 (CN)
(72) Inventor: ZHOU, Yali, Nanjing Jiangsu 210061 (CN); CHEN, Gong, Nanjing Jiangsu 210061 (CN); JIANG, Xiaoyan, Nanjing Jiangsu 210061 (CN); REN, Jiangtao, Nanjing, Jiangsu 210061 (CN); HE, Xiaohong, Nanjing, Jiangsu 210061 (CN); WANG, Yanbin, Nanjing, Jiangsu 210061 (CN); HAN, Lu, Nanjing Jiangsu 210061 (CN)
(74) Representative: Comoglio, Elena
(86) International application number: PCT/CN2021/113520
(87) International publication number: WO 2022/022745

(57) **Abstract**

A chimeric antigen receptor, containing a ligand binding domain, a transmembrane domain, a co-stimulatory domain, and an intracellular signaling domain, the co-stimulatory domain containing CD94 and/or LTβ intracellular regions. The present invention further relates to engineered immune cells containing such a chimeric antigen receptor, and uses thereof in the treatment of diseases, such as cancer, autoimmune diseases, and infections.

## Description

### Technical Field

The present disclosure belongs to the field of immunotherapy. More specifically, the present disclosure relates to a chimeric antigen receptor comprising a novel co-stimulatory domain, and an engineered immune cell comprising such chimeric antigen receptor and use thereof.

### Background Art

In recent years, cancer immunotherapy technology has developed rapidly, and especially, chimeric antigen receptor T cell (CAR-T)-related immunotherapy has achieved excellent clinical results in the treatment of hematological tumors. CAR-T cell immunotherapy is to genetically modify T cells in vitro so that they can recognize tumor antigens, and after amplified to a certain number, they are infused back into the patient's body to kill cancer cells, thereby achieving the purpose of treating tumors.

At present, with the development of technology, four generations of different CAR structures have emerged. The intracellular signaling domain of the first-generation CAR only contains a primary signaling domain, such as CD3ζ, therefore CAR-carrying cells (such as CAR-T cells) have poor activity and short survival time in vivo. The second-generation CAR, in which a co-stimulatory domain, such as CD28 or 4-1 BB, is introduced, enabling cells to proliferate continuously with enhanced anti-tumor activity. The third-generation CAR contains two co-stimulatory domains (such as CD28+4-1 BB), and in the fourth-generation CAR, cytokines or co-stimulatory ligands are included to further enhance T cell responses, or suicide genes are included to achieve self-destruction of CAR-T cells when needed. Most of the current clinical researches still use the second-generation CAR structure.

However, there are still some problems in the clinical application of CAR-T cell therapy, for example, there are a large number of tumor recurrences in the treatment of hematological tumors, and the response rate is low in the treatment of solid tumors, and so on, which may be caused by complex tumor microenvironment, CAR-T cell exhaustion and other factors.

Therefore, there is still a need to improve the existing CAR T cell therapy to promote the proliferation of CAR T cells in vivo, resist the immunosuppressive effect of the tumor microenvironment, and thus improving the overall therapeutic effect of CAR T cell therapy on tumors.

### Summary

Therefore, in a first aspect, the present disclosure provides a chimeric antigen receptor, comprising a ligand binding domain, a transmembrane domain, a co-stimulatory domain and an intracellular signaling domain, wherein the co-stimulatory domain comprises a CD94 intracellular region and/or an LTβ intracellular region.

In an embodiment, the CD94 intracellular region has at least 90%, 95%, 97% or 99%, or 100% sequence identity to the amino acid sequence represented by SEQ ID NO: 25, and the LTβ intracellular region has at least 90%, 95%, 97% or 99%, or 100% sequence identity to the amino acid sequence represented by SEQ ID NO: 27.

In an embodiment, the co-stimulatory domain further comprises the signaling domain of a protein selected from the group consisting of: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, ZAP70, and combinations thereof. Preferably, the co-stimulatory domain further comprises the signaling domain of CD27, CD28, CD134, CD137 or CD278 or a combination thereof, more preferably further comprises the signaling domain of CD28 and/or CD137.

In an embodiment, the ligand binding domain is an antibody or an antigen binding portion thereof.

In an embodiment, the ligand binding portion is selected from the group consisting of whole antibody, Fab, Fab', F(ab')2, Fv fragment, scFv antibody fragment, linear antibody, sdAb or nanobody.

In an embodiment, the ligand binding domain binds to a target selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D and any combination thereof. Preferably, the ligand binding domain binds to a target selected from the group consisting of: CD19, CD20, CD22, CD30, CD33, CD38, CD123, CD138, CD171, MUC1, AFP, Folate receptor α, CEA, PSCA, PSMA, Her2, EGFR, IL13Ra2, GD2, NKG2D, EGFRvIII, CS1, BCMA, mesothelin, and any combination thereof.

In an embodiment, the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CDS α, CD9, CD16, CD22, CD33, CD28, CD37, CD64, CD80, CD86, CD134, CD137, and CD154.

In an embodiment, the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d, preferably CD3ζ signaling domain.

In a second aspect, the present disclosure further provides a nucleic acid comprising a sequence encoding the chimeric antigen receptor of the present disclosure, a vector comprising the nucleic acid, and an immune cell comprising the nucleic acid or the vector.

In an embodiment, the disclosure provides a nucleic acid comprising a sequence encoding the chimeric antigen receptor of the present disclosure. Preferably, the nucleic acid is DNA or RNA, more preferably DNA.

In an embodiment, the present disclosure provides a vector comprising the above nucleic acid. Specifically, the vector is selected from the group consisting of linear nucleic acid molecule, plasmid, retrovirus, lentivirus, adenovirus, vaccinia virus, Rous Sarcoma Virus (RSV), polyoma virus, and adeno-associated virus (AAV), bacteriophage, phagemid, cosmid or artificial chromosome. In some embodiments, the vector further contains elements such as an origin of self-replication in an immune cell, a selectable marker, a restriction enzyme cleavage site, a promoter, a poly-A tail (polyA), 3' UTR, 5' UTR, an enhancer, a terminator, an insulator, an operon, a selectable marker, a reporter gene, a targeting sequence, and/or a protein purification tag. In a specific embodiment, the vector is an *in vitro* transcription vector.

In an embodiment, the present disclosure provides an immune cell comprising the nucleic acid or vector of the disclosure capable of expressing the chimeric antigen receptor of the disclosure. In a specific embodiment, the immune cell is selected from the group consisting of a T cell, a macrophage, a dendritic cell, a monocyte, a NK cell or a NKT cell. Preferably, the T cell is a CD4+/CD8+ double positive T cell, a CD4+ helper T cell, a CD8+ T cell, a tumor infiltrating cell, a memory T cell, a naive T cell, a γδ-T cell or an αβ-T cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell.

In a third aspect, the present disclosure provides a pharmaceutical composition, comprising the chimeric antigen receptor of the present disclosure or the encoding nucleic acid thereof, vector or immune cell comprising the same, and one or more pharmaceutically acceptable excipients.

In a fourth aspect, the present disclosure further provides a method of treating cancer, infection or autoimmune disease, including administering to the subject an effective amount of the chimeric antigen receptor, the engineered immune cell or the pharmaceutical composition according to the present disclosure.

The present disclosure further provides the use of the chimeric antigen receptor, nucleic acid, vector, engineered immune cell or pharmaceutical composition as defined above in the preparation of a medicine for treating cancers, infections or autoimmune diseases.

In an embodiment, the cancer is selected from the group consisting of: brain glioma, blastoma, sarcoma, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (including gastrointestinal cancer), glioblastoma (GBM), liver cancer, hepatoma, intraepithelial tumor, kidney cancer, larynx cancer, liver tumor, lung cancer (e.g., small cell lung cancer, non-small cell lung cancer, lung adenocarcinoma and squamous lung cancer), melanoma, myeloma, neuroblastoma, oral cancer (e.g., lips, tongue, mouth, and pharynx), ovarian cancer, pancreatic cancer, prostate cancer, mesothelioma, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer, Waldenstrom macroglobulinemia, lymphoma (including Hodgkin's lymphoma and non-Hodgkin's lymphoma, such as B cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, Burkitt lymphoma, diffuse large B cell lymphoma, follicular lymphoma, MALT lymphoma, marginal zone lymphoma, plasmablastic lymphoma, plasmacytoid dendritic cell tumor), leukemia (including acute leukemia such as acute lymphoblastic leukemia, acute myelogenous leukemia, acute nonlymphocytic leukemia such as acute myeloblastic leukemia (including undifferentiated and partially differentiated), acute promyelocytic leukemia, acute myelomonocytic leukemia, acute monocytic leukemia, erythroleukemia, acute megakaryoblastic leukemia; chronic leukemia such as chronic myelogenous leukemia, chronic lymphocytic leukemia, chronic monocytic leukemia; and other special types of leukemia such as hairy cell leukemia, prolymphocytic leukemia, plasma cell leukemia, adult T-cell leukemia, eosinophilic leukemia, basophilic leukemia, etc.), blastic plasmacytoid dendritic cell tumor, malignant lymphoproliferative disease, myeloid dysplasia, multiple myeloma, myelodysplasia, and post-transplant lymphoproliferative disorder (PTLD).

In an embodiment, the infection includes, but is not limited to, infections caused by viruses, bacteria, fungi, and parasites.

In an embodiment, the autoimmune disease includes, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

The advantage of the present disclosure is that the signaling domain from CD94 and/or LTβ as a new co-stimulatory domain can provide better co-stimulatory activity, enhance the survival and persistence of CAR-modified engineered immune cell, thereby enhancing the inhibitory effect of CAR cell on tumors.

### Detailed Description of Embodiments

Unless otherwise indicated, all scientific and technical terms used herein have the same meaning as commonly understood by one of ordinary skill in the art to which the present disclosure belongs.

### Chimeric antigen receptor

As used herein, the term "chimeric antigen receptor" or "CAR" refers to an artificially constructed hybrid polypeptide, where the hybrid polypeptide generally includes a ligand binding domain (e.g., an antibody or an antigen-binding portion thereof), a transmembrane domain, a co-stimulatory domain, and an intracellular signaling domain, with various domains linked via a linker. CAR can redirect the specificity and reactivity of T cell and other immune cells to a selected target in a non-MHC-restricted manner by utilizing the antigen binding properties of antibodies. Non-MHC-restricted antigen recognition gives CAR-expressing immune cells an antigen recognition ability independent of antigen processing, thus bypassing the major mechanism of tumor escape.

In a first aspect, the present disclosure provides a chimeric antigen receptor, comprising a ligand binding domain, a transmembrane domain, a co-stimulatory domain and an intracellular signaling domain, wherein the co-stimulatory domain comprises a CD94 intracellular region and/or an LTβ intracellular region.

A costimulatory domain is an intracellular functional signaling domain from a costimulatory molecule, which contains the entire intracellular portion of the costimulatory molecule, or a functional fragment thereof. A "costimulatory molecule" refers to a cognate binding partner that specifically binds to a costimulatory ligand on a T cell, thereby mediating a costimulatory response (e.g., proliferation) of the T cell. Traditional chimeric antigen receptors comprise CD28 or 4-1 BB as a co-stimulatory domain. The chimeric antigen receptor of the disclosure comprises a novel costimulatory domain, i,e., intracellular region from CD94 or LTβ. The present disclosure finds that the addition of CD94 intracellular region and/or LTβ intracellular region as a novel co-stimulatory domain can significantly increase the expansion level of CAR cells, thereby improving the inhibitory effect on tumors.

CD94 is a type II transmembrane protein and a member of the C-type lectin family. CD94 consists of 180 amino acids, in which the extracellular domain contains two N-linked glycosylation sites. The extracellular region of CD94 contains four interchain disulfide bonds, in which Cys61-Cys72, Cys89-Cys174 and Cys152-Cys156 are the characteristic disulfide bonds of C-type lectin receptors, and the disulfide bond between Cys59-Cys70 is a unique structure of CD94. The Cys58, the only one that does not form a disulfide bond in the CD94 molecule, forms an interchain disulfide bond with the corresponding cysteine residue in the NKG2 family molecule, thereby forming a CD94/NKG2 heterodimer. The NKG2 family includes NKG2A, NKG2B, NKG2C, NKG2D, NKG2E, NKG2F and other members, in which NKG2A, NKG2B, NKG2C and NKG2E can bind to CD94. CD94/NKG2A (or NKG2B, as an alternatively spliced form of NKG2A) is an inhibitory receptor that can recognize the non-classical MHC-class I molecule HLA-E and the antigenic peptide presented thereby, and transmit inhibitory signals to NK cells through the two immunoreceptor tyrosine inhibitory motifs (ITIM) contained in the intracellular region of NKG2A. CD94/NKG2C and CD94/NKG2E are activating receptors, and can bind to the adapter molecule DAP12 through the lysine residues in the transmembrane region, therefore phosphorylating the tyrosine residues in the immunoreceptor tyrosine-based activation motif (ITAM) contained in the intracellular region of the latter, recruiting ZAP-70 and Syk, and leading to NK cell activation.

It is generally believed that the intracellular region of CD94 is so short that it does not have the function of signal transmission. However, the inventors unexpectedly discovered that the intracellular domain of CD94 can act as a co-stimulatory domain to transmit signals when used in a chimeric antigen receptor. Therefore, in an embodiment, the chimeric antigen receptor of the present disclosure comprises the intracellular region of CD94 as a co-stimulatory domain, which has at least 70%, preferably at least 80%, more preferably at least 90%, 95%, 97% or 99% or 100% sequence identity to an amino acid sequence represented by SEQ ID NO: 25, or an encoding sequence thereof has at least 70%, preferably at least 80%, more preferably at least 90%, 95%, 97% or 99% or 100% sequence identity to a nucleotide sequence represented by SEQ ID NO: 26.

LTβ, also known as LTB, lymphotoxin β or p33, is widely expressed on the surface of T cells, NK cells, CD4+ CTL cells, B cells, lymphokine-activated killer (LAK) cells, and various tumor cell lines, such as various T lymphoma cells, B cells in chronic lymphocytic leukemia, and so on. LTβbinds with another subunit LTαto form a heterotrimeric complex, LTα1β2 or LTα2β1, and the entire complex is anchored to the cell membrane through the hydrophobic amino acids contained in LTβ. In the absence of LTβ, LTα is released out of cells. Studies have shown that LTβ alone cannot be properly aggregated, and can only exert corresponding biological functions when assembled with LTα to form a heterotrimeric complex. LTβ exerts its biological functions by binding to its specific receptor LTβR, such as cytotoxicity, induction of cell apoptosis, promotion of cell proliferation, regulation of immune system development and so on.

The inventors discovered for the first time that the intracellular region of LTβ can serve as a co-stimulatory domain to provide stimulation signals. Therefore, in an embodiment, the chimeric antigen receptor of the present disclosure comprises an LTβ intracellular region as a co-stimulatory domain, which has at least 70%, preferably at least 80%, more preferably at least 90%, 95%, 97% or 99% or 100% sequence identity to an amino acid sequence represented by SEQ ID NO: 27, or an encoding sequence thereof has at least 70%, preferably at least 80%, more preferably at least 90%, 95%, 97% or 99% or 100% sequence identity to a nucleotide sequence represented by SEQ ID NO: 28.

In addition to the novel CD94 and LTβ co-stimulatory domains provided by the present disclosure, the chimeric antigen receptors of the present disclosure may further comprise one or more additional co-stimulatory domains selected from the co-stimulatory signaling domains of the following proteins: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, or ZAP70, preferably selected from 4-1BB, CD28, CD27, OX40 or combinations thereof, more preferably selected from 4-1BB, CD28.

In an embodiment, the CAR of the disclosure comprises a CD94 intracellular region as a co-stimulatory domain. In an embodiment, the CAR of the disclosure comprises an LTβ intracellular region as a co-stimulatory domain. In an embodiment, the CAR of the disclosure comprises CD94 and 4-1BB co-stimulatory domains. In an embodiment, the CAR of the disclosure comprises LTβ and 4-1BB co-stimulatory domains. In an embodiment, the CAR of the disclosure comprises CD94 and CD28 co-stimulatory domains. In an embodiment, the CAR of the disclosure comprises LTβ and CD28 co-stimulatory domains.

4-1BB and CD28 co-stimulatory domains are known to those skilled in the art. For example, the 4-1BB co-stimulatory domain has at least 70%, preferably at least 80%, more preferably at least 90%, 95%, 97% or 99% or 100% sequence identity to an amino acid sequence represented by SEQ ID NO: 9, or an encoding sequence thereof has at least 70%, preferably at least 80%, more preferably at least 90%, 95%, 97% or 99% or 100% sequence identity to a nucleotide sequence represented by SEQ ID NO: 10. The CD28 co-stimulatory domain has at least 70%, preferably at least 80%, more preferably at least 90%, 95%, 97% or 99% or 100% sequence identity to an amino acid sequence represented by SEQ ID NO: 7, or an encoding sequence thereof has at least 70%, preferably at least 80%, more preferably at least 90%, 95%, 97% or 99% or 100% sequence identity to a nucleotide sequence represented by SEQ ID NO: 8.

As used herein, "ligand binding domain" refers to any structure or functional variant thereof that can bind to a ligand (e.g. antigen). The ligand binding domain may be an antibody structure, including, but not limited to, monoclonal antibody, polyclonal antibody, recombinant antibody, human antibody, humanized antibody, murine antibody, chimeric antibody, and antigen-binding fragment thereof. For example, the ligand binding domain includes, but is not limited to, whole antibody, Fab, Fab', F(ab')2, Fv fragment, scFv antibody fragment, linear antibody, sdAb (VH or VL), nanobody (Nb), recombinant fibronectin domain, anticalin, DARPIN, and so on, preferably selected from Fab, scFv, sdAb, and nanobody. In the present disclosure, the ligand binding domain may be monovalent or bivalent, and may be a monospecific, bispecific or multispecific antibody.

"Fab" refers to any one of two identical fragments produced after an immunoglobulin molecule is cleaved by papain, and consists of an intact light chain and a heavy chain N-terminal part linked by a disulfide bond, wherein the heavy chain N-terminal part includes a heavy chain variable region and CH1. Compared with intact IgG, Fab has no Fc fragment, has relatively high fluidity and tissue penetration ability, and can univalently bind to an antigen without mediating antibody effects.

"Single chain antibody" or "scFv" is an antibody composed of antibody's heavy chain variable region (VH) and light chain variable region (VL) linked by a linker. The optimal length and/or amino acid composition of the linker can be selected. The length of the linker will significantly affect the variable region folding and interaction of the scFv. In fact, intrachain folding can be prevented if a shorter linker (e.g. 5-10 amino acids) is used. Regarding the selection of size and composition of the linker, see, e.g., Hollinger et al., 1993 Proc Natl Acad. Sci. U.S.A. 90:6444-6448; US patent applications with publication Nos. 2005/0100543, 2005/0175606, 2007/0014794; and PCT applications with publication Nos. WO2006/020258 and WO2007/024715, which are incorporated herein by reference in their entirety. The scFv may contain a VH and a VL linked in any order, e.g., VH-linker-VL or VL-linker-VH.

"Single domain antibody" or "sdAb" refers to an antibody that naturally lacks light chain, and the antibody contains only one heavy chain variable region (VHH) and two conventional CH2 and CH3 regions, also known as "heavy chain antibody".

"Nanobody" or "Nb" refers to a VHH structure that is individually cloned and expressed, which has structural stability and binding activity to an antigen comparable to those of the original heavy chain antibody, and is the smallest unit currently known to be capable of binding to a target antigen.

The term "functional variant" or "functional fragment" refers to a variant that substantially includes the amino acid sequence of a parent, but, compared with the parent amino acid sequence, contains at least one amino acid modification (i.e., substitution, deletion, or insertion), provided that the variant retains the biological activity of the parent amino acid sequence. For example, for an antibody, a functional fragment thereof is the antigen-binding portion thereof. In an embodiment, the amino acid modification is preferably a conservative modification.

As used herein, the term "conservative modification" refers to amino acid modification that does not significantly affect or alter the binding characteristics of the antibody or antibody fragment containing the amino acid sequence. These conservative modifications include amino acid substitution, addition, and deletion. The modifications can be introduced into the chimeric antigen receptor of the present disclosure by standard techniques known in the art, such as site-directed mutagenesis and PCR-mediated mutagenesis. The conservative amino acid substitution is a substitution in which the amino acid residue is replaced with an amino acid residue having a similar side chain. Amino acid residue families having a similar side chain have been defined in the art, including basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), nonpolar side chain (e.g., alanine, valine, leucine, isoleucine, proline, phenylalanine, methionine, tryptophan), β-branched side chain (e.g., threonine, valine, isoleucine), and aromatic side chain (e.g., tyrosine, phenylalanine, tryptophan, histidine). The conservative modifications may be selected, for example, based on polarity, charge, solubility, hydrophobicity, hydrophilicity, and/or similarity in amphiphilic properties of residues involved.

Thus, the "functional variant" or "functional fragment" has at least 75%, preferably at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the parent amino acid sequence, and retains the biological activity, e.g., binding activity, of the parent amino acid.

As used herein, the term "sequence identity" indicates the degree to which two (nucleotide or amino acid) sequences have the same residue at the same position in an alignment, and is generally expressed by percentage. Preferably, the identity is determined over the entire length of the sequences being compared. Thus, two copies with completely identical sequences have 100% identity. Those skilled in the art will recognize that some algorithms can be used to determine sequence identity using standard parameters, for example, Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197), and ClustalW.

The selection of ligand binding domain depends on the cell surface marker on a target cell to be recognized and associated with a specific disease state, for example, a tumor specific antigen or a tumor associated antigen. Thus, in an embodiment, the ligand binding domain of the present disclosure binds to one or more targets selected from the group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, Claudin18.2, NKG2D, and any combination thereof. Preferably, the target is selected from the group consisting of: CD19, CD20, CD22, BAFF-R, CD33, EGFRvIII, BCMA, GPRC5D, PSMA, ROR1, FAP, ERBB2 (Her2/neu), MUC1, EGFR, CAlX, WT1, NY-ESO-1, CD79a, CD79b, GPC3, Claudin18.2, NKG2D, and any combination thereof. Depending on the antigen to be targeted, the CAR of the present disclosure may be designed to include a ligand binding domain specific for the antigen. For example, if CD19 is the antigen to be targeted, a CD19 antibody can be used as a ligand binding domain of the present disclosure.

In an embodiment, the chimeric antigen receptor of the present disclosure targets CD19, CD22, or a combination thereof. In a preferred embodiment, the chimeric antigen receptor of the present disclosure comprises an anti-CD19 antibody, which comprises a light chain variable region sequence having at least 70%, preferably at least 80%, more preferably at least 90%, 95%, 97%, 99% or 100% sequence identity to an amino acid sequence represented by positions 1-107 of SEQ ID NO: 1 or positions 1-107 of SEQ ID NO: 29, and a heavy chain variable region sequence having at least 70%, preferably at least 80%, more preferably at least 90%, 95%, 97%, 99% or 100% sequence identity to an amino acid sequence represented by positions 123-242 of SEQ ID NO: 1 or positions 123-238 of SEQ ID NO: 29. In a preferred embodiment, the chimeric antigen receptor of the present disclosure comprises an anti- CD22 antibody, which comprises a heavy chain variable region sequence having at least 70%, preferably at least 80%, more preferably at least 90%, 95%, 97%, 99% or 100% sequence identity to an amino acid sequence represented by positions 1-124 of SEQ ID NO: 31, and a light chain variable region sequence having at least 70%, preferably at least 80%, more preferably at least 90%, 95%, 97%, 99% or 100% sequence identity to an amino acid sequence represented by positions 143-249 of SEQ ID NO: 31.

As used herein, the term "transmembrane domain" refers to a polypeptide structure that enables expression of a chimeric antigen receptor on the surface of an immune cell (e.g., a lymphocyte, an NK cell, or an NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric antigen receptor binds to the target antigen. The transmembrane domains particularly suitable for use in the present disclosure may be derived from, for example, a TCR α chain, a TCR β chain, a TCR γ chain, a TCR δ chain, a CD3 ζ subunit, a CD3 ε subunit, a CD3 γ subunit, a CD3 δ subunit, CD45, CD4, CD5, CDS α, CD9, CD16, CD22, CD33, CD28, CD37, CD64, CD80, CD86, CD134, CD137, CD154, and functional fragments thereof. Alternatively, the transmembrane domain may be synthesized and may mainly contain a hydrophobic residue such as leucine and valine. Preferably, the transmembrane domain is derived from a CDS α chain or CD28, which has at least 70%, preferably at least 80%, more preferably at least 90%, 95%, 97% or 99% or 100% sequence identity to an amino acid sequence represented by SEQ ID NO: 3 or 5, or an encoding sequence of thereof has at least 70%, preferably at least 80%, more preferably at least 90%, 95%, 97% or 99% or 100% sequence identity to a nucleotide sequence represented by SEQ ID NO: 4 or 6.

In an embodiment, the chimeric antigen receptor of the present disclosure further may contain a hinge region located between the ligand binding domain and the transmembrane domain. As used herein, the term "hinge region" generally refers to any oligopeptide or polypeptide that functions to link a transmembrane domain to a ligand binding domain. Specifically, the hinge region serves to provide greater flexibility and accessibility to the ligand binding domain. The hinge region may contain up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region contains a hinge region portion of a CD8 α chain, an Fc γ RIII α receptor, CD28, an IgG4, or an IgG1, more preferably a hinge from CD8α, CD28 or IgG4, which has at least 70%, preferably at least 80%, more preferably at least 90%, 95%, 97% or 99% or 100% sequence identity to an amino acid sequence represented by SEQ ID NO: 19, 21 or 23, or an encoding sequence thereof has at least 70%, preferably at least 80%, more preferably at least 90%, 95%, 97% or 99% or 100% sequence identity to a nucleotide sequence represented by SEQ ID NO: 20, 22 or 24.

As used herein, the term "intracellular signaling domain" refers to a protein portion that transduces an effector function signal and guides a cell to perform a specified function. The intracellular signaling domain is responsible for intracellular primary signaling after the ligand binding domain binds to the antigen, thus causing activation of immune cell and immune reaction. In other words, the intracellular signaling domain is responsible for activating at least one of the normal effector functions of the immune cells in which the CAR is expressed. For example, the effector functions of T cell can be cytolytic activity or auxiliary activity, including secretion of cytokines.

In an embodiment, the intracellular signaling domain contained in the chimeric antigen receptor of the present disclosure may be cytoplasmic sequences of a T cell receptor and a co-receptor, upon antigen receptor binding, which act together to initiate primary signaling, as well as any derivative or variant of these sequences and any synthetic sequence having the same or similar function. The intracellular signaling domain may contain many immunoreceptor tyrosine-based activation motifs (ITAM). Non-limiting examples of intracellular signaling domain of the present disclosure include, but are not limited to, those derived from FcR γ, FcR β, CD3 γ, CD3 δ, CD3 ε, CD3 ζ, CD22, CD79a, CD79b, and CD66d. In a preferred embodiment, the signaling domain of the CAR of the present disclosure may contain a CD3 ζ signaling domain, and the signaling domain has at least 70%, preferably at least 80%, more preferably at least 90%, 95%, 97% or 99%, or 100% sequence identity to an amino acid sequence represented by SEQ ID NO: 11 or 13, or an encoding sequence thereof has at least 70%, preferably at least 80%, more preferably at least 90%, 95%, 97% or 99%, or 100% sequence identity to a nucleotide sequence represented by SEQ ID NO: 12 or 14.

In an embodiment, the co-stimulatory domain and the intracellular signaling domain can be operably linked in any order. For example, the co-stimulatory domain can be located on the proximal end of the membrane, while the intracellular signaling domain is located on the distal end of the membrane. Alternatively, the stimulatory domain is located on the distal end of the membrane, and the intracellular signaling domain is located on the proximal end of the membrane. When two or more co-stimulatory domains are contained, the co-stimulatory domains may be located on one or both sides of the intracellular signaling domain.

In an embodiment, the CAR of the present disclosure further may contain a signal peptide such that when it is expressed in a cell such as a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment recognized and cleaved by signal peptidase. The signal peptidase can cleave during or after translocation, so as to generate free signal peptide and mature protein. Then, the free signal peptide is digested by a specific protease. Signal peptides that can be used in the present disclosure are well known to those skilled in the art, for example, signal peptides derived from CD8 α, IgG1, GM-CSFRα, B2M, and so on. In an embodiment, the signal peptide that can be used in the present disclosure has at least 70%, preferably at least 80%, more preferably at least 90%, 95%, 97% or 99%, or 100% sequence identity to an amino acid sequence represented by SEQ ID NO: 15 or 17, or an encoding sequence of the signal peptide has at least 70%, preferably at least 80%, more preferably at least 90%, 95%, 97% or 99%, or 100% sequence identity to a nucleotide sequence represented by SEQ ID NO: 16 or 18.

In an embodiment, the CAR of the present disclosure further may contain a switch structure to regulate the expression time of the CAR. For example, the switch structure may be in a form of dimerization domain, which causes a conformational change by binding to a corresponding ligand thereof, and exposes the extracellular binding domain to enable its binding to a targeted antigen, thereby activating a signaling pathway. Alternatively, a switch domain also may be used to link the binding domain and signaling domain, respectively, and only when the switch domains are bound to each other (for example, in the presence of an inducing compound), the binding domain and the signaling domain can be linked together through a dimer, thereby activating signaling pathway. The switch structure also can be in the form of a masking peptide. The masking peptide can shield the extracellular binding domain, and prevent it from binding to the targeted antigen. When the masking peptide is cleaved by, for example, a protease, the extracellular binding domain is exposed, making it become a "normal" CAR structure. A variety of switch structures known to those skilled in the art can be used in the present disclosure.

In an embodiment, the CAR of the present disclosure further may contain a suicide gene, to make it express a cell death signal that can be induced by an exogenous substance, so as to eliminate the CAR cell when needed (e.g., when serious toxic side effects are produced). For example, the suicide gene may be in the form of an inserted epitope, e.g., a CD20 epitope, an RQR8, etc., and when needed, the CAR cell can be eliminated by adding an antibody or reagent that targets these epitopes. The suicide gene also may be herpes simplex virus thymidine kinase (HSV-TK), which gene can induce the cell to die when receiving ganciclovir treatment. The suicide gene further may be iCaspase-9, and dimerization of iCaspase-9 can be induced by a chemical induction drug such as AP1903 and AP20187, so as to activate the downstream Caspase3 molecule, and cause apoptosis. A variety of suicide genes known to those of skill in the art can be used in the present disclosure.

### Nucleic acid

The present disclosure further provides a nucleic acid molecule, which contains a nucleic acid sequence encoding the chimeric antigen receptor of the present disclosure.

As used herein, the term "nucleic acid molecule" includes a sequence of ribonucleotide and deoxyribonucleotide, such as modified or unmodified RNA or DNA, each in single-stranded and/or double-stranded form, linear or circular, or their mixtures (including hybrid molecules). Thus, the nucleic acid according to the present disclosure includes DNA (e.g. dsDNA, ssDNA, cDNA), RNA (e.g. dsRNA, ssRNA, mRNA, ivtRNA), their combinations or derivatives (e.g. PNA). Preferably, the nucleic acid is DNA or RNA, more preferably mRNA.

The nucleic acid may contain a conventional phosphodiester bond or an unconventional bond (e.g., amide bond, such as found in peptide nucleic acid (PNA)). The nucleic acid of the present disclosure further may contain one or more modified bases, such as, for example, trityl base and uncommon base (such as inosine). Other modifications also can be contemplated, including chemical, enzymatic, or metabolic modifications, so long as the multi-chain CAR of the present disclosure can be expressed from polynucleotides. The nucleic acid can be provided in isolated form. In an embodiment, the nucleic acid also may include a regulatory sequence, such as a transcriptional control element (including a promoter, an enhancer, an operon, a repressor, and a transcription termination signal), ribosome binding sites, and introns.

The nucleic acid sequences of the present disclosure can be codon-optimized for optimal expression in a desired host cell (e.g., immune cell); or for expression in a bacterial, yeast, or insect cell. Codon optimization refers to substitution of a codon in the target sequence that is generally rare in highly expressed genes of a given species with a codon that is generally common in highly expressed genes of such species, and the codons before and after the substitution encode the same amino acid. Therefore, the selection of an optimal codon depends on the codon usage preference of the host genome.

### Vector

The present disclosure further provides a vector, containing the nucleic acid molecule of the present disclosure.

As used herein, the term "vector" is an intermediary nucleic acid molecule used to transfer (exogenous) genetic material into a host cell, and in the host cell the nucleic acid molecule can be, for example, replicated and/or expressed.

The vector generally includes targeting vectors and expression vectors. The "targeting vector" is a medium that delivers an isolated nucleic acid to the interior of a cell by, for example, homologous recombination or by using a hybrid recombinase of a sequence at specific target site. The "expression vector" is a vector used for transcription of heterologous nucleic acid sequences (for example, those sequences encoding the chimeric antigen receptor polypeptides of the present disclosure) in suitable host cells and the translation of their mRNAs. Suitable vectors that can be used in the present disclosure are known in the art, and many are commercially available. In an embodiment, the vector of the present disclosure includes, but is not limited to, plasmid, virus (e.g., retrovirus, lentivirus, adenovirus, vaccinia virus, Rous sarcoma virus (RSV), polyoma virus, and adeno-associated virus (AAV), etc.), bacteriophage, phagemid, cosmid, and artificial chromosome (including BAC and YAC). The vector itself is usually a nucleotide sequence, and usually is a DNA sequence containing an insert (transgene) and a larger sequence as "backbone" of the vector. Engineered vector typically also contains an origin autonomously replicating in the host cell (if stable expression of polynucleotide is desired), a selectable marker, and a restriction enzyme cleavage site (e.g., a multiple cloning site, MCS). The vectors may additionally contain elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an *in vitro* transcription vector.

### Engineered immune cell

The present disclosure further provides an engineered immune cell, which comprises the chimeric antigen receptor or the encoding nucleic acid thereof.

As used herein, the term "immune cell" refers to any cell of the immune system that has one or more effector functions (e.g., cytotoxic cell killing activity, secretion of cytokines, induction of ADCC and/or CDC). Immune cells can be obtained from a variety of sources, including peripheral blood mononuclear cells, bone marrow, lymph node tissue, cord blood, thymus tissue, tissue from sites of infection, ascites, pleural effusion, spleen tissue, and tumors. Immune cells can also be derived from stem cells such as adult stem cells, embryonic stem cells, cord blood stem cells, progenitor cells, bone marrow stem cells, induced pluripotent stem cells, totipotent stem cells, or hematopoietic stem cells. For example, the immune cell may be a T cell, a macrophage, a dendritic cell, a monocyte, an NK cell, and/or an NKT cell. Preferably, the immune cell is a T cell. The T cell may be any T cell, such as *in vitro* cultured T cell, for example, primary T cell, or T cell from *in vitro* cultured T cell line, e.g., Jurkat, SupT1, etc., or T cell obtained from a subject. Examples of a subject include humans, dogs, cats, mice, rats, and transgenic species thereof. The T cell also may be concentrated or purified. The T cell may be a T cell of any type and at any stage of development, including, but not limited to, a CD4+/CD8+ double positive T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell, etc. In a preferred embodiment, the immune cell is a human T cell, a NK cell or a NKT cell. The T cell can be obtained from the blood of a subject using a variety of techniques known to those of skill in the art, such as Ficoll isolation. In the present disclosure, the immune cell is engineered to express the chimeric antigen receptor polypeptide.

The nucleic acid sequence encoding the chimeric antigen receptor polypeptide can be introduced into an immune cell using conventional methods known in the art (e.g., by transduction, transfection, transformation), so that the chimeric antigen receptor polypeptide of the present disclosure can be expressed. "Transfection" is a process of introducing a nucleic acid molecule or polynucleotide (including a vector) into a target cell. An example is RNA transfection, i.e., the process of introducing RNA (such as *in vitro* transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation can be achieved by artificial means. In order for transformation to occur, the cell or bacterium must be in a competent state. For prokaryotic transformation, the techniques may include heat shock-mediated uptake, fusion to bacterial protoplasts of intact cells, microinjection, and electroporation.

In an embodiment, the immune cell of the present disclosure may further comprise suppressed or silenced expression of at least one gene selected from the group consisting of CD52, GR, TCR α, TCR β, CD3 γ, CD3 δ, CD3 ε, CD247 ζ, HLA-I, HLA-II gene, and immune checkpoint gene such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3. More particularly, at least TCR α or TCR β gene, and/or HLA-I or HLA-II in immune cells are inactivated. This strategy is particularly useful for avoiding or reducing the risk of immune rejection. Methods for inhibiting or silencing gene expression are known in the art. For example, antisense RNA, RNA decoy, RNA aptamer, siRNA, shRNA/miRNA, trans dominant negative protein (TNP), chimeric/fusion protein, chemokine ligand, anti-infective cellular protein, intrabody (sFv), nucleoside analog (NRTI), non-nucleoside analog (NNRTI), integrase inhibitor (oligonucleotide, dinucleotide, and chemical agent) and protease inhibitor may be used to inhibit gene expression. Alternatively, genes can also be silenced by DNA fragmentation mediated by for example meganucleases, zinc finger nucleases, TALE nucleases or Cas enzymes in CRISPR systems.

### Pharmaceutical composition

The present disclosure further provides a pharmaceutical composition, which contains the chimeric antigen receptor, the nucleic acid, the vector or the engineered immune cell described in the present disclosure as an active agent, and one or more pharmaceutically acceptable excipients. Therefore, the present disclosure further encompasses use of the chimeric antigen receptor, the nucleic acid, the vector or the engineered immune cell in the preparation of a pharmaceutical composition or medicine.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, cosolvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the application is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicine may include, for example, conventional mixing, dissolving, granulating, dragee-making, grinding, emulsifying, encapsulating, embedding or lyophilizing process. The pharmaceutical composition containing, for example, the immune cell as described herein is generally provided in a form of solution, and preferably contains a pharmaceutically acceptable buffer agent.

The pharmaceutical composition according to the present disclosure further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210 and 213, actinides 225 and astatine 213; prodrugs such as antibody-directed enzyme prodrugs; immunostimulatory agents such as IL-2, chemokines such as IL-8, platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Method for preparing engineered immune cells

The present disclosure further provides a method for preparing an engineered immune cell, comprising introducing the chimeric antigen receptor of the present disclosure or nucleic acid sequence encoding it into an immune cell, so that the immune cell expresses the chimeric antigen receptor of the present disclosure.

In an embodiment, the immune cell is a human immune cell, more preferably a human T cell, a macrophage, a dendritic cell, a monocyte, a NK cell and/or a NKT cell.

Methods for introducing nucleic acids or vectors into immune cells for expression are known in the art. For example, nucleic acids or vectors may be introduced into immune cells by physical methods, such as calcium phosphate precipitation, lipofection, particle bombardment, microinjection, electroporation, and so on. Alternatively, nucleic acids or vectors may be introduced into immune cells by chemical methods, such as through colloidal dispersion systems, such as macromolecular complexes, nanocapsules, microspheres, beads, and lipid-based systems, including oil-in-water emulsions, micelles, mixed micelles, and liposomes. In addition, nucleic acids or vectors can also be introduced using biological methods. For example, viral vectors, especially retroviral vectors, etc. have become the most common method for inserting genes into mammalian, e.g. human cells. Other viral vectors can be derived from lentiviruses, poxviruses, herpes simplex virus I, adenoviruses, adeno-associated viruses, and so on.

After introducing the nucleic acid or vector into the immune cell, a person skilled in the art could amplify and activate the resulting immune cell by conventional techniques.

### Therapeutic use

The present disclosure further provides a method of treating a subject with cancer, infection or autoimmune disease, including administering to the subject an effective amount of the chimeric antigen receptor, the nucleic acid molecule, the vector, the engineered immune cell or the pharmaceutical composition according to the present disclosure. Therefore, the present disclosure further encompasses use of the chimeric antigen receptor, the nucleic acid molecule, the vector or the engineered immune cell in the preparation of a medicine for treating cancer, infection, or autoimmune diseases.

In an embodiment, an effective amount of the immune cell and/or the pharmaceutical composition of the present disclosure is directly administered to the subject.

In another embodiment, the treatment method of the present disclosure is *ex vivo* treatment. Specifically, the method includes the steps of: (a) providing a sample from a subject, the sample containing an immune cell; (b) introducing the chimeric antigen receptor of the present disclosure into the immune cell *in vitro* to obtain a modified immune cell, and (c) administering the modified immune cell to the subject in need thereof. Preferably, the immune cell provided in step (a) is selected from a macrophage, a dendritic cell, a monocyte, a T cell, an NK cell, and/or an NKT cell; and the immune cell can be obtained from the sample (particularly a blood sample) of the subject by conventional methods known in the art. However, other immune cells capable of expressing the chimeric antigen receptor and exogenous gene of the present disclosure and exerting the desired biological effect function as described herein also can be used. Besides, the immune cells generally selected are compatible with the subject's immune system, i.e., it is preferred that the immune cells do not trigger an immunogenic response. For example, a "universal recipient cell", i.e., a universally compatible lymphocyte exerting a desired biological effect function and being capable of growing and amplifying *in vitro,* can be used. The use of such cells will not require obtaining and/or providing the subject's own lymphocyte. The *ex vivo* introduction of step (b) may be carried out by introducing the nucleic acid or vector described herein into the immune cell via electroporation or by infecting the immune cell with a viral vector, wherein the viral vector is a lentiviral vector, adenoviral vector, adeno-associated viral vector or retroviral vector as previously described. Other conceivable methods include using a transfection reagent (such as a liposome) or transient RNA transfection.

In an embodiment, the immune cell is an autologous or allogeneic cell, preferably T cell, macrophage, dendritic cell, monocyte, NK cell and/or NKT cell, more preferably T cell, NK cell or NKT cell.

As used herein, the term "autologous" means that any material derived from an individual will be later re-introduced into the same individual.

As used herein, the term "allogeneic" means that the material is derived from a different animal or different patient of the same species as the individual into which the material is introduced. When the genes at one or more loci are different, two or more individuals are considered allogeneic to each other. In some cases, genetic differences in allogeneic material from various individuals of the same species may be sufficient for antigen interactions to occur.

As used herein, the term "subject" refers to a mammal. The mammal may be, but is not limited to, a human, a non-human primate, a mouse, a rat, a dog, a cat, a horse, or a cow. Mammals other than human can be advantageously used as subjects representing cancer animal models. Preferably, the subject is a human.

In an embodiment, the cancer is a cancer associated with expression of the target to which the ligand binding domain binds. For example, the cancer includes, but is not limited to, brain glioma, blastoma, sarcoma, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (including gastrointestinal cancer), glioblastoma (GBM), liver cancer, hepatoma, intraepithelial tumor, kidney cancer, larynx cancer, liver tumor, lung cancer (e.g., small cell lung cancer, non-small cell lung cancer, lung adenocarcinoma and squamous lung cancer), melanoma, myeloma, neuroblastoma, oral cancer (e.g., lips, tongue, mouth, and pharynx), ovarian cancer, pancreatic cancer, prostate cancer, mesothelioma, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer, Waldenstrom macroglobulinemia, lymphoma (including Hodgkin's lymphoma and non-Hodgkin's lymphoma, such as B cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, Burkitt lymphoma, diffuse large B cell lymphoma, follicular lymphoma, MALT lymphoma, marginal zone lymphoma, plasmablastic lymphoma, plasmacytoid dendritic cell tumor), leukemia (including acute leukemia such as acute lymphoblastic leukemia, acute myelogenous leukemia, acute nonlymphocytic leukemia such as acute myeloblastic leukemia (including undifferentiated and partially differentiated), acute promyelocytic leukemia, acute myelomonocytic leukemia, acute monocytic leukemia, erythroleukemia, acute megakaryoblastic leukemia; chronic leukemia such as chronic myelogenous leukemia, chronic lymphocytic leukemia, chronic monocytic leukemia; and other special types of leukemia such as hairy cell leukemia, prolymphocytic leukemia, plasma cell leukemia, adult T-cell leukemia, eosinophilic leukemia, basophilic leukemia, etc.), blastic plasmacytoid dendritic cell tumor, malignant lymphoproliferative disease, myeloid dysplasia, multiple myeloma, myelodysplasia, and post-transplant lymphoproliferative disorder (PTLD); and other diseases associated with target expression. Preferably, the disease which can be treated with the engineered immune cell or the pharmaceutical composition of the present disclosure is selected from the group consisting of: leukemia, lymphoma, multiple myeloma, brain glioma, pancreatic cancer, ovarian cancer, mesothelioma, breast cancer, lung cancer, prostate cancer, melanoma, myeloma, sarcoma, gastric cancer, and so on.

In an embodiment, the infection includes, but is not limited to, infections caused by viruses, bacteria, fungi, and parasites.

In an embodiment, the autoimmune disease includes, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

In an embodiment, the method further includes administering to the subject one or more additional chemotherapeutic agents, biological agents, medicines, or treatments. In this embodiment, the chemotherapeutic agents, biological agents, medicines, or treatments are selected from the group consisting of radiotherapy, surgery, antibody reagent and/or small molecule and any combination thereof.

The present disclosure will be described in detail below with reference to the accompanying drawings and examples. It should be noted that those skilled in the art should understand that the accompanying drawings of the present disclosure and examples thereof are only for illustrative purpose, and cannot constitute any limitation to the present disclosure. The examples of the present disclosure and the features in the examples may be combined with each other without contradiction.

### Brief Description of Drawings

Fig. 1 shows structural schematic view of the chimeric antigen receptor of the present disclosure.
Fig. 2 shows scFv expression levels in z-CAR, 94z-CAR and LTBz-CAR T cells.
Fig. 3 shows the expansion levels of CAR-T cells expressing z-CAR, 94z-CAR and LTBz-CAR.
Fig. 4 shows scFv expression levels in CAR-T cells expressing bbz-CAR, bbz94-CAR and bbzLTB-CAR.
Fig. 5 shows the killing effect of CAR-T cells expressing bbz-CAR, bbz94-CAR and bbzLTB-CAR on target cells at concentrations of various effector-to-target ratios.
Fig. 6 shows the cytokine release levels of CAR-T cells expressing bbz-CAR, bbz94-CAR and bbzLTB-CAR.
Fig. 7 shows the in vivo expansion levels of CAR-T cells expressing bbz-CAR, bbz94-CAR and bbzLTB-CAR.
Fig. 8 shows the survival rate of tumor-bearing mice in each group treated with CAR-T cells.

### Specific Embodiments

The T cells used in all the examples of the present disclosure are primary human CD4+CD8+ T cells isolated from healthy donors by leukapheresis using Ficoll-Paque^{™} PREMIUM (GE Healthcare, Cat. No. 17-5442-02).

### Example 1. Preparation of CAR T cells

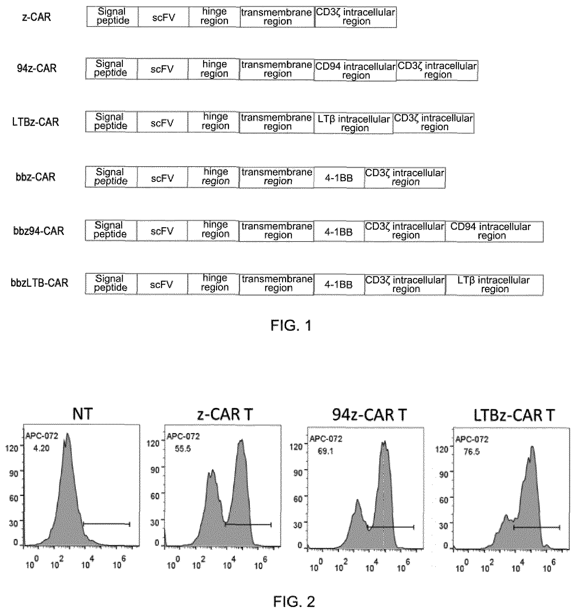

The following coding sequences were synthesized and cloned into pGEM-T Easy vector (Promega, Cat. No. A1360) to prepare CAR constructs: CD8α signal peptide (SEQ ID NO: 18), anti-CD19 scFv (SEQ ID NO: 2), CD8α hinge region (SEQ ID NO: 20), CD8α transmembrane region (SEQ ID NO: 4), costimulatory domain, CD3ζ intracellular signaling domain (SEQ ID NO: 12), wherein the costimulatory domain is absent (z-CAR), CD94 intracellular region (SEQ ID NO: 26, 94z-CAR) or LTβ intracellular region (SEQ ID NO: 28, LTBz-CAR), and the correct insertion of the target sequence was confirmed by sequencing. The structure of CAR is shown in Figure 1.

After the above plasmids were diluted by adding 3ml Opti-MEM (Gibco, Cat. No. 31985-070) in a sterile tube, the packaging vector psPAX2 (Addgene, Cat. No. 12260) and the envelope vector pMD2.G (Addgene, Cat. No. 12259) were added according to the ratio of plasmid : viral packaging vector: viral envelope vector = 4:2:1. Then, 120ul X-treme GENE HP DNA transfection reagent (Roche, Cat. No. 06366236001) was added, mixed immediately, and incubated at room temperature for 15min. Then, the plasmid/vector/transfection reagent mixture was added dropwise to the culture flask of 293T cells. Viruses were collected at 24 hours and 48 hours, pooled, and ultracentrifuged (25000g, 4°C, 2.5 hours) to obtain concentrated lentiviruses.

T cells were activated with DynaBeads CD3/CD28 CTSTM (Gibco, Cat. No. 40203D) and cultured at 37°C and 5% CO2 for 1 day. Then, the concentrated lentivirus was added, and after continuous culture for 3 days, the traditional z-CAR T cells targeting CD19 and the 94z-CAR T and LTBz-CAR T cells of the present disclosure were obtained. Unmodified wild-type T cells were used as negative controls (NT).

After culturing at 37°C and 5% CO2 for 11 days, Biotin-SP (long spacer) AffiniPure Goat Anti-Mouse IgG, F(ab')₂ Fragment Specific (min X Hu, Bov, Hrs Sr Prot) (jackson immunoresearch , Cat. No. 115-065-072) were used as the primary antibody, and APC Streptavidin (BD Pharmingen, Cat. No. 554067) or PE Streptavidin (BD Pharmingen, Cat. No. 554061) was used as the secondary antibody, and the expression level of scFv on CAR-T cells was detected by flow cytometry. The results are shown in Figure 2.

It can be seen that the scFv in the CAR T cells prepared by the present disclosure can be effectively expressed.

### Example 2: In vitro expansion level of CAR-T cells

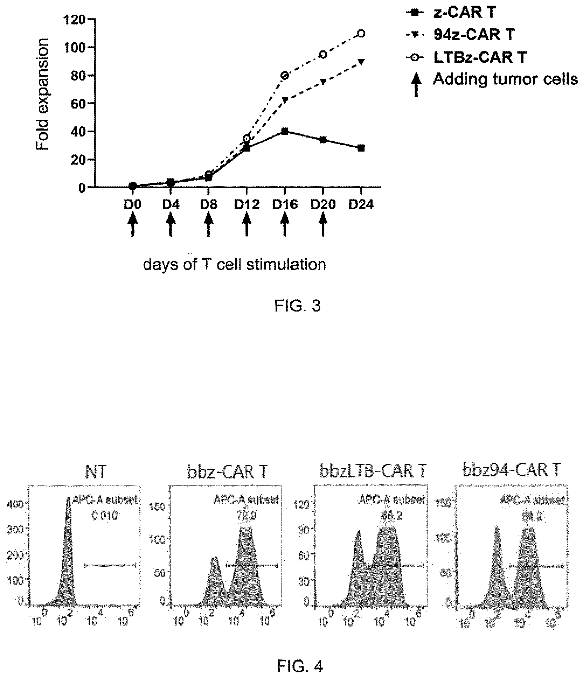

In order to determine the expansion ability of CAR-T cells in vitro, Nalm6 target cells carrying the green fluorescent protein gene were first plated in a 24-well plate at 1×10⁶/well, and then, CAR-T cells expressing 94z-CAR, LTBz-CAR or z-CAR were plated into the 24-well plate at an effector-to-target ratio (i.e., the ratio of effector T cells to target cells) of 1:1 for co-culture (D0), and on D4, D8, D12, D16, and D20, the target cells were further added at an effector-to-target ratio of 1:1. The number of expanded CAR T cells was counted regularly, and the results are shown in Figure 3.

It can be seen that the expansion level of z-CAR T cells without co-stimulatory domain gradually decreased after D12, while 94z-CAR and LTBz-CAR T cells could continue to expand, with an in vitro expansion level after D12 significantly higher than that of the z-CAR T group. This indicates that the CD94 intracellular region and the LTβ intracellular region can serve as co-stimulatory domains to efficiently provide stimulating signals to T cells, thereby increasing the expansion level of CAR-T cells.

### Example 3: Killing effect of CAR T cells on target cells and release of cytokines

### 3.1 Preparation of CAR-T cells

The following coding sequences were synthesized and cloned into pGEM-T Easy vector (Promega, Cat. No. A1360) to prepare CAR constructs: CD8α signal peptide (SEQ ID NO: 18), anti-CD19 scFv (SEQ ID NO: 30), CD8α hinge region (SEQ ID NO: 20), CD8α transmembrane region (SEQ ID NO: 4), 4-1BB co-stimulatory domain (SEQ ID NO: 10), CD3ζ intracellular signaling domain (SEQ ID NO: 12), and the correct insertion of the target sequence was confirmed by sequencing to obtain bbz-CAR.

An additional costimulatory domain CD94 intracellular region (SEQ ID NO: 26) or LTβ intracellular region (SEQ ID NO: 28) was further added to bbz-CAR to obtain bbz94-CAR and bbzLTB-CAR, respectively. The structure of CAR is shown in Figure 1.

According to the method described in Example 1, flow cytometry was used to detect the expression level of scFv in the above CAR-T cells, and the results are shown in FIG. 4.

It can be seen that the scFv in the bbz-CAR, bbz94-CAR and bbzLTB-CAR T cells prepared by the present disclosure can all be effectively expressed.

### 3.2 The killing effect of CAR-T cells on target cells

When T cells kill target cells, the number of target cells decreases. After co-culturing T cells with target cells expressing luciferase, the number of target cells decreases and the secretion of luciferase decreases accordingly. Luciferase can catalyze the conversion of luciferin to oxidized luciferin, and during this oxidation process, bioluminescence will be generated, and the intensity of this luminescence will depend on the level of luciferase expressed by the target cells. Therefore, the detected fluorescence intensity can reflect the ability of T cells to kill target cells.

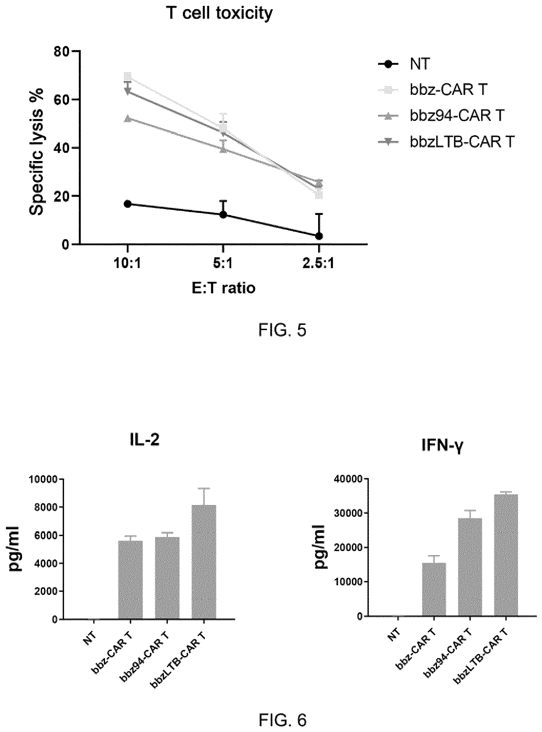

In order to detect the killing ability of CAR-T cells on target cells, Nalm6 target cells carrying the fluorescein gene were first plated into a 96-well plate at 1×10⁴/well, and then cells expressing bbz94-CAR, bbzLTB-CAR or bbz-CAR and NT cells were plated into the 96-well plate at an effector-to-target ratio (i.e. the ratio of effector T cells to target cells) of 10:1, 5:1 or 2.5:1, respectively for co-culture. After 16-18 hours, a microplate reader was used to measure the fluorescence value. The killing efficiency was calculated according to the calculation formula: (average fluorescence value of target cells - average fluorescence value of samples)/average fluorescence value of target cells × 100%, and the results are shown in Figure 5.

It can be seen that compared with NT, the specific killing effect of the CAR T cells of the present disclosure on target cells is equivalent to that of traditional bbz-CAR T cells at various concentrations of effector-target ratios.

### 3.3 Cytokine release of CAR-T cells

When T cells kill target cells, the number of target cells decreases, and at the same time, cytokines IL-2 and IFN-γ are released. According to the following steps, enzyme-linked immunosorbent assay (ELISA) was used to measure the release levels of cytokines IL-2 and IFN-γ when target cells were killed by CAR T cells.

### (1) Collection of supernatant of the cell co-culture

Target cells Nalm6 were plated in a 96-well plate at a concentration of 1×10⁵/well, and then bbz94-CAR, bbzLTB-CAR, bbz-CAR T cells and NT cells were co-cultured with target cells at a ratio of 1:1, and the supernatants of the cell co-cultures were collected after 18-24 hours.

### (2) ELISA detection of the secretion of IL-2 and IFN-γ in the supernatant

Purified anti-human IL2 Antibody (Biolegend, Cat. No. 500302) or Purified anti-human IFN-γ Antibody (Biolegend, Cat. No. 506502) were used as capture antibody to coat the 96-well plate by incubating overnight at 4°C. After removing the antibody solution, 250 µL PBST (1XPBS containing 0.1% Tween) containing 2 % BSA (sigma, Cat. No. V900933-1kg) was added and incubated at 37°C for 2 hours. Plates were then washed 3 times with 250 µL PBST (1XPBS containing 0.1% Tween). 50 µL of cell co-culture supernatant or standards per well were added and incubated at 37 °C for 1 h, then the plate was washed 3 times with 250 µL of PBST (1XPBS with 0.1% Tween). Then 50 µL Anti-Interferon gamma antibody [MD-1] (Biotin) (abeam, Cat. No. ab25017) as detection antibody was added to each well and after incubating at 37°C for 1 hour, the plate was washed 3 times with 250 µL PBST (1XPBS containing 0.1% Tween). Then HRP Streptavidin (Biolegend, Cat. No. 405210) was added, and incubated at 37°C for 30 minutes. The supernatant was discarded, and 250 µL PBST (1XPBS containing 0.1% Tween) was added for washing 5 times. 50 µL of TMB substrate solution was added to each well. Reactions were allowed to occur at room temperature in the dark for 30 minutes, and then 50 µL of 1 mol/L H₂SO₄ was added to each well to stop the reaction. Within 30 minutes after the reaction was stopped, the absorbance at 450nm was detected using a microplate reader, and the content of cytokines was calculated according to the standard curve (drawn according to the reading value and concentration of the standard), and the results are shown in Figure 6.

It can be seen that the release of IL-2 and IFN-γ was not detected in the NT group, indicating that the killing of target cells by bbz-CAR T cells and the CAR T cells of the present disclosure is specific. In addition, compared with traditional bbz-CAR T cells, the release level of IL-2 in bbz94-CAR T cells did not change significantly, but the release level of IFN-γ was significantly higher; while the release levels of IL-2 and IFN-γ of bbzLTB-CAR T cells were higher than those of bbz-CAR T group. Therefore, in general, the cytokine release levels of bbz94-CAR T cells and bbzLTB-CAR T cells of the present disclosure are higher than those of traditional bbz-CAR T cells.

### Example 3 Verification of the tumor suppressive effect of CAR-T cells

The suppressive effect of CAR-T cells on tumors was verified in a mouse model.

Twenty 8-week-old healthy female NCG mice were divided into four groups: NT group (negative control), bbz-CAR T group, bbz94-CAR T group, and bbzLTB-CAR T group. On day 0 (D0), 1×10⁶ Nalm6 cells were injected intravenously into the tail of each mouse. Seven days later (D7), each mouse was injected intravenously with PBS solution or 2×10⁶ NT cells, bbz-CAR T cells, bbz94-CAR T cells or bbzLTB-CAR T cells at the tail according to the grouping.

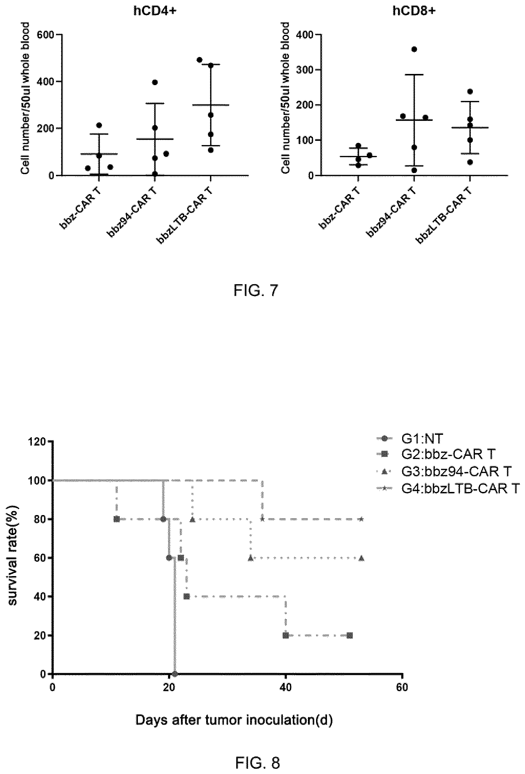

On D21, submandibular vein blood was taken from mice in the bbz-CAR T, bbz94-CAR T and bbzLTB-CAR T groups, and the expression levels of hCD8 and hCD4 were analyzed by Trucount FACS to detect the expansion of CAR T cells in the mice. The results are shown in Figure 7.

It can be seen that T cell expansion was detected in all CAR T group mice tested, and the expansion of CD4+ T cells and CD8+ T cells in bbz94-CAR T group and bbzLTB-CAR T group was significantly higher than that in bbzCAR T group.

In addition, the inventors also counted the survival percentage of mice in each group by the end of the experiment (i.e., day 53 after inoculation of tumor cells Nalm6) (FIG. 8). All the mice in the NT group died on day 21, only one mouse (20%) treated with bbz-CAR T cells survived, and 3 mice treated with bbz94-CAR T cells survived ( accounted for 60%), and 4 mice survived in the bbzLTB-CAR T group with a survival rate as high as 80%. This shows that the addition of CD94 and LTβ co-stimulatory domains can significantly improve the inhibitory effect of CAR-T cells on tumors and improve the survival rate of tumor-bearing mice.

In summary, compared with traditional CAR T cells, the CAR-T cells containing CD94 intracellular region and LTβ intracellular region in the present disclosure can greatly promote the expansion of T cells due to the introduction of a new co-stimulatory domain structure, so as to improve the sustained killing effect on tumor cells and improve the tumor suppression effect.

## Claims

1. A chimeric antigen receptor, comprising a ligand binding domain, a transmembrane domain, a co-stimulatory domain and an intracellular signaling domain, **characterized in that** the co-stimulatory domain comprises a CD94 intracellular region and/or an LTβ intracellular region.

2. The chimeric antigen receptor according to claim 1, **characterized in that** the CD94 intracellular region has at least 90%, 95%, 97% or 99%, or 100% sequence identity to an amino acid sequence represented by SEQ ID NO: 25, and the LTβ intracellular region has at least 90%, 95%, 97% or 99%, or 100% sequence identity to an amino acid sequence represented by SEQ ID NO: 27.

3. The chimeric antigen receptor according to claim 1 or 2, **characterized in that** the co-stimulatory domain further comprises a signaling domain of a protein selected from the group consisting of: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, ZAP70, and combinations thereof.

4. The chimeric antigen receptor according to claim 1 or 2, **characterized in that** the co-stimulatory domain further comprises a signaling domain of CD27, CD28, CD134, CD137 or CD278 or a combination thereof.

5. The chimeric antigen receptor according to claim 1 or 2, **characterized in that** the ligand binding domain is an antibody or an antigen binding portion thereof.

6. The chimeric antigen receptor according to claim 5, **characterized in that** the antigen binding portion is selected from the group consisting of Fab, Fab', F(ab')2, Fv fragment, scFv antibody fragment, linear antibody, sdAb or nanobody.

7. The chimeric antigen receptor according to any one of claims 1-6, **characterized in that** the ligand binding domain binds to a target selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D and any combination thereof.

8. The chimeric antigen receptor according to any one of claims 1-7, **characterized in that** the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137 and CD154.

9. The chimeric antigen receptor according to any one of claims 1-8, **characterized in that** the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d.

10. A nucleic acid molecule, encoding the chimeric antigen receptor according to any one of claims 1-9.

11. A vector, comprising the nucleic acid molecule according to claim 10.

12. An engineered immune cell, comprising the chimeric antigen receptor according to any one of claims 1-9 or the nucleic acid molecule according to claim 10.

13. The engineered immune cell according to claim 12, **characterized in that** the immune cell is selected from the group consisting of a T cell, a macrophage, a dendritic cell, a monocyte, a NK cell or a NKT cell.

14. The engineered immune cell according to claim 13, **characterized in that** the T cell is a CD4+/CD8+ double positive T cell, a CD4+ helper T cell, a CD8+ T cell, a tumor infiltrating cell, a memory T cell, a naive T cell, a γδ-T cell or an αβ-T cell.

15. The engineered immune cell of claims 12-14, **characterized in that** the immune cell is derived from an adult stem cell, an embryonic stem cell, a cord blood stem cell, a progenitor cell, a bone marrow stem cell, an induced pluripotent stem cell, a totipotent stem cell, or a hematopoietic stem cell.

16. A pharmaceutical composition, comprising the engineered immune cell according to any one of claims 1-15, and one or more pharmaceutically acceptable excipients.

17. Use of the chimeric antigen receptor according to any one of claims 1-9, the nucleic acid molecule according to claim 10, the vector according to claim 11 or the engineered immune cell according to any one of claims 12-16 in preparation of a medicine for treating cancers, infections or autoimmune diseases.
